Europäisches Patentamt

European Patent Office

Office européen des brevets

(19)

(11) Publication number: 0 122 537

A1

(12) EUROPEAN PATENT APPLICATION

(21) Application number: 84103686.6

(22) Date of filing: 04.04.84

(51) Int. Cl.³: G 02 B 23/08
A 61 B 1/04

(30) Priority: 11.04.83 JP 63469/83
11.04.83 JP 53822/83
17.05.83 JP 85045/83

(43) Date of publication of application:
24.10.84 Bulletin 84/43

(84) Designated Contracting States:
DE FR GB IT NL SE

(71) Applicant: SUMITOMO ELECTRIC INDUSTRIES LIMITED
No. 15, Kitahama 5-chome Higashi-ku
Osaka-shi Osaka-fu(JP)

(72) Inventor: Ono, Kimizo c/o Osaka Works of Sumitomo
Electric Ind., Ltd. 1-3, Shimaya 1-chome
Konohana-ku Osaka(JP)

(72) Inventor: Iwamoto, Tomio c/o Osaka Works of
Sumitomo
Electric Ind., Ltd. 1-3, Shimaya 1-chome
Konohana-ku Osaka(JP)

(72) Inventor: Matsuki, Syuji c/o Osaka Works of Sumitomo
Electric Ind., Ltd. 1-3, Shimaya 1-chome
Konohana-ku Osaka(JP)

(74) Representative: Glawe, Delfs, Moll & Partner
Patentanwälte
Postfach 26 01 62 Liebherrstrasse 20
D-8000 München 26(DE)

(54) Device for remote viewing.

(57) A device for observing pictures of an object is proposed which has an image transmission line (11) for transmitting an image, an optical system (10) for forming the image of the object at the front end of the image transmission line (11), a reflex mirror (2) for reflecting the light from the object and directing it toward the optical system (10), a flexible cable (3) accommodating the image transmission line (11), and an image receiving means (14) provided at the other end of the image transmission line for observing the image transmitted, the image transmission line (11) having an image fiber comprising a bundle of picture element fibers.

FIG.3

0122537

# DEVICE FOR OBSERVING PICTURES

The present invention relates to a device for observing pictures used for the observation of confined portions such as an oral cavity in dental or other medical treatments. This device is also applicable for industrial use such as for the maintenance of equipment, and for civil engineering and construction work.

A drawback contingent to the observation of an image reflected in a mirror is that the mirror image is reversed bilaterally from the actual image. The inspection of an oral cavity conducted by a dentist or a nose, ear and throat specialist will be taken as an example to explain this drawback.

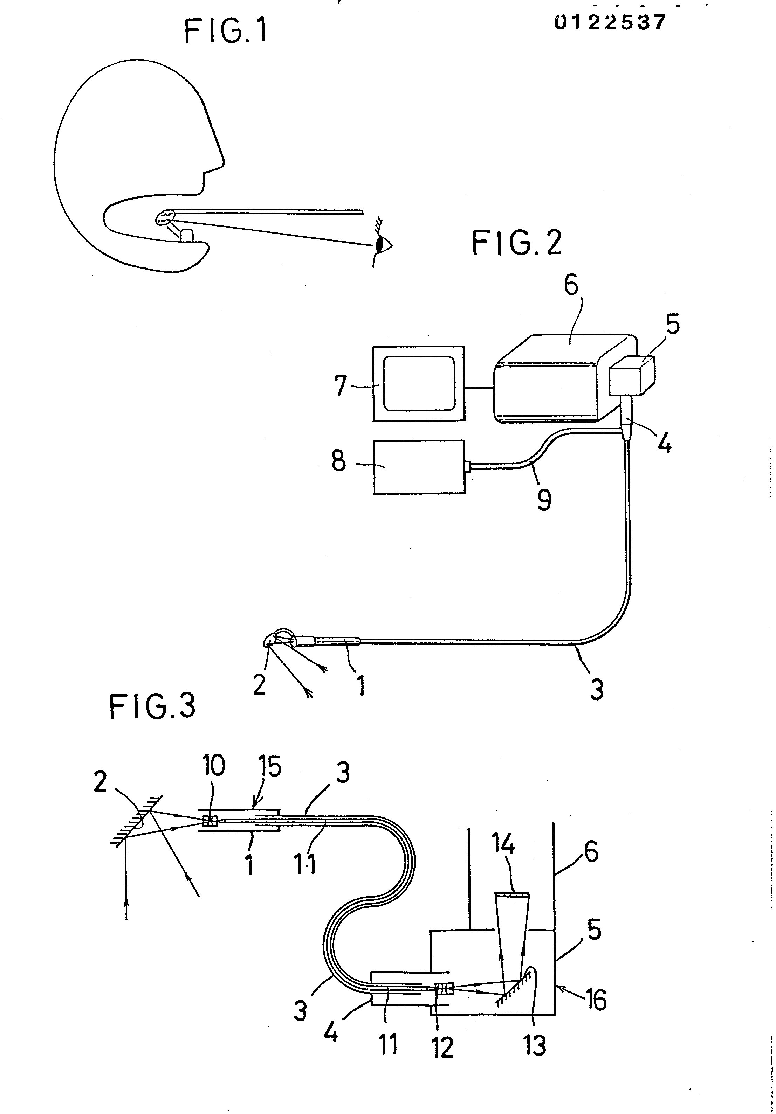

As shown in Fig. 1, a bar with a reflex mirror mounted on its end with an angle of inclination has been used to inspect an oral cavity. It permits the inspection of a portion which cannot be seen directly in the oral cavity, such as the back side of a tooth, because the light from the back side of the tooth is reflected by the reflex mirror and comes into the eye of a dentist. This appliance is easy to handle because it is light in weight and small in size. Therefore, with this appliance an oral cavity can be inspected easily.

However, this method has the following disadvantage:

- 1 -

(1)   An image is bilaterally reversed as mentioned above.

(2)   The object is not so well lit.  In other words, it is often hard to see an object because of dimness.

(3)   It is difficult to see a narrow region.  Because the conventional appliance has no means for magnifying an object, you have to bring the eye close to the oral cavity of a patient for detailed inspection of a narrow region.  However, there is a limit in doing so.

(4)   The affected part cannot be inspected by a plurality of persons at a time, but only by a single dentist.  The patient cannot see the affected part with his own eyes.  Therefore, he cannot receive treatment with his understanding and consent.  A plurality of physicians cannot inspect the same seat of a disease at a time to discuss about the diagnosis and treatment.

Such a bilateral reversal does not embarrass a person so much when he inspects with such a simple appliance as shown in Fig. 1.  However, when a dentist inspects an oral cavity by means of a large-scale observation device, it will be strange to look at a bilaterally reversed image projected on a large screen of a video monitor.  It will be natural that he wishes an image not reversed on the screen.

It is an object of the present invention to provide a device for observing pictures which obviates the above-
described disadvantages.

The device for observing pictures in accordance
with the present invention comprises an image transmission
line, an optical system for forming the image of an object
at the front end of the image transmission line, a reflex
mirror for reflecting the light from the object to direct
it toward the optical system, a flexible cable having
the image transmission line therein, and an image-receiving
optical system provided at the rear end of the image
transmission line for the inspection of the transmitted
image, wherein the image transmission line is provided
by an image fiber comprising a bundle of picture element
fibers.  A feature of the present invention is that by
reflecting an image in an even number of times, no bilateral
reversal will occur.

Another feature of the present invention is that
the reflex mirror used is not in a perfectly circular
form but in the form of an elongated circle with its
minor axis at a right angle to the image transmission
line.

The above-described and other objects and features
of this invention will be readily appreciated as the
same becomes better understood by reference to the following detailed description when considered in connection with the accompanying drawings, in which:

Fig. 1 is a view illustrating a conventional device of inspecting an oral cavity;

Fig. 2 is a schematic view of the entire device in accordance with the present invention;

Fig. 3 is a schematic view of an embodiment in which a reflex mirror is added in the image-receiving unit;

Fig. 4 is a longitudinal section of a probe used in the device in accordance with the present invention;

Fig. 5 is a view taken in the direction of arrows V-V of Fig. 4;

Fig. 6 is a sectional view taken along line VI-VI of Fig. 4;

Fig. 7 is a sectional view taken along line VII-VII of Fig. 4;

Fig. 8 is a schematic view of the end of the probe and the mirror;

Fig. 9 is a view showing how the surface of the mirror is divided into utilizable (blank) and unutilizable (hatched) areas;

Fig. 10 is a view explaining how a curved line is shaped when the cone formed by lights coming into the end of the image fiber intersects a plane which forms an angle of $\theta$ therewith; and

Fig. 11 is a view explaining the terms elongated circle, elliptical, and perfectly circular;

Referring now to Fig. 2, a probe 1 accommodates an image fiber for transmitting an image and a light guide for transmitting the light for illumination, and an optical system for forming the image of an object being inspected at the end of the image fiber. A reflex mirror 2 is mounted in front of the probe 1 at an angle on the extension of the optical axis of the image fiber. The light for illumination is projected from the light guide and reflected by the reflex mirror 2 to illuminate the object. The light from the object is reflected by the reflex mirror 2 and focused by a lens at the end of the image fiber. The image fiber and the light guide run parallel with each other in a flexible cable 3 and are connected to an image-receiving adapter 4.

The image transmitted by the image fiber is picked up by a TV camera 6 through an optical system 5 coupled therewith, and is projected on the screen of a TV monitor 7.

The light from a light source 8 for illumination, such as a halogen lamp or a xenon lamp, is transmitted through a light guide 9 and the light guide in the flexible cable 3 and reaches the object via the reflex mirror 2. Instead of being projected on the screen of the TV monitor 7, the image picked up by the TV camera 6 may be recorded in a video tape recorder.

A feature of the present invention is that an even number of reflex mirrors are used to reflect the light from the object in the even number of times before an image comes to a viewer.

This can be done in the following three manners:

(1) Another mirror is added between the reflex mirror 2 and the probe 1. The light from the object is reflected by the two mirrors so that the image incident on the end of the probe will not be a reversed one.

(2) An optical device for the reversal of an image, which includes an odd number of reflex mirrors and an optical system, is added in the intermediate portion of the flexible cable 3. Such an optical device may comprise, e.g., a lens and a prism or prisms serving as reflex mirrors.

(3) Another reflex mirror is added in the image-receiving unit.

In short, one or an odd number of reflex mirrors may be added in a suitable position in the light path.

Fig. 3 illustrates an embodiment in which a reflex mirror for the reversal of an image is added in the image-receiving unit. The light guide is omitted. No light guide has to be provided when some other means for supplying illumination light is available. Only an image pickup unit 15 and an image-receiving unit 16 are illustrated.
A lens assembly 10 is provided to form the image of the
object at the front end of an image fiber 11.

The image transmitted through the image fiber 11
is bilaterally reversed.  In the image-receiving unit
16, a lens 12 and a reflex mirror 13 are provided in
the optical system 5 coupled with the TV camera 6.  The
image reflected by the reflex mirror 13 is incident on
a surface 14 of the TV camera 6.  The reflex mirror 13
is a newly added optical element.  The image, which has
already been bilaterally reversed, is further subjected
to a bilateral reversal by the mirror 13 back to the
actual shape of the object.

The reflex mirror 13 changes the direction in which
the light travels.  Therefore, the image-receiving adapter
4 is connected to the TV camera 6 so that the axis of
the former will be at a right angle to the axis of the
latter.

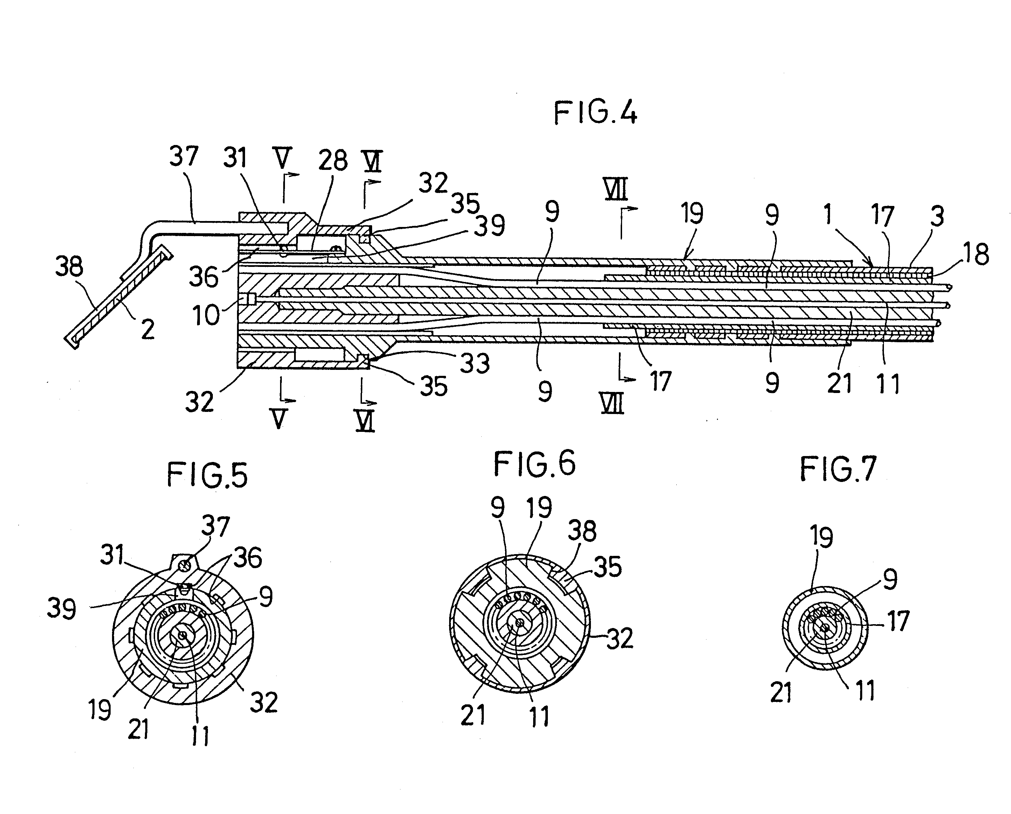

Referring to Figs. 4-7, the image fiber cable has
a flexible cable 3, an image fiber 11 accommodated therein,
a covering tube 21 for protecting the image fiber, a
plurality of optical fibers 9 for illumination disposed
around the covering tube 21, a pliable resin covering
17, a helically wound flexible protecting cover 18 made
of metal, a resin flexible covering 3 surrounding the protecting cover 18, and a stator 19 fitting on the end
of the flexible cable 3 and forming a grip by which the
device is held, e.g., by a dentist.

The narrow portion of the stator 19 fits on the
armored end of the image fiber cable 1. The stator is
a hollow tube made of stainless steel. At its end, the
end of the image fiber 11 and the ends of the optical
fibers 9 for illumination are open. The end of the image
fiber 11 is open through a vacant space and a lens assembly
10.

Because of the armoring structure of the image fiber
cable, the angle of torsion allowed for the image fiber
cable and the stator 19 is not so large. That is why
a rotor 32 made of stainless steel is mounted on the
stator 19 in the proximity of its end. The rotor 32
is adapted to be turnable around the axis of the stator
19 and is prevented from slipping out of the stator.
The rotor 32 carries a reflex mirror 2 by which the light
reflected from the object is reflected to the open end
of the image fiber 11 disposed at the end of the stator
19.

While rotating around the axis of the stator, the
rotor 32 is adapted to stop intermittently as mentioned
below. Therefore, the rotor 32 can be stopped in a desired
position so that the position of the reflex mirror 2 relative to the image fiber will be fixed.  One can do
this work while watching the TV pictures.  In order to
change the visual field during the work, the rotor 32
is released by turning the rotor 32 in a desired direction.
The rotor is moved to the next stop position where the
work can be resumed.  Thus the reflex mirror can be freely
swiveled and fixed at such a position as to afford excellent work efficiency.

The rotor 32 is mounted on and in sliding contact
with the stator.  The inner surface of the rotor is provided
with axial grooves 36 at regular intervals.  A stop pin
31 mounted on a leaf spring 28, one end of which is secured
to the stator 19, falls in one of the grooves 36. (Fig.
5)

The lens assembly 10 is mounted in front of the
end of the image fiber 11 to afford a larger aperture
angle.  The reflex mirror 2 forms an angle of inclination
with the axis of the stator 19 so as to facilitate inspection of the back side of an object.

Each time the rotor 32 makes a revolution by 360
degrees around the axis of the stator 19, axial grooves
38 provided in the stator 19 are aligned with projections
35 asymmetrically provided on the rotor 32 so that the
rotor can be axially removed from the stator 19. (Fig.
6)  Therefore, after the device in accordance with the present invention has been used for dental treatment, the rotor 32 is turned around the axis of the stator until the axial grooves 38 come into alignment with the projections 35. Then the rotor is axially pulled and detached from the stator. The rotor and the stator are now ready for disinfection and washing.

By turning the rotor 32 against the resilience of the leaf spring 28, the reflex mirror 2 can be placed in position where the object is easily inspected. Since the rotor is adapted to turn intermittently, it can be held at one of the stop positions on the stator 19 so that the reflex mirror will be prevented from getting out of position relative to the image fiber during inspection.

A mirror mounting rod 37 projects frontwardly from the end of the end cover 32. A mirror mount 38 is welded to the mirror mounting rod 37. A mirror 2 is secured to the mirror mount 38. The center of the mirror 2 lies on the extension of the image fiber 11. However, the axis of the image fiber has not necessarily to be exactly aligned with the center of the mirror. The surface of the mirror forms an angle of $\theta$ with the axis of the image fiber. Although $\theta$ is equal to 45° in this embodiment, it may not be 45°.

What is important is that the mirror 2 is elliptical. The minor axis of this elliptical mirror is perpendicular to the axis of the image fiber, while its major axis forms an angle of θ with the axis of the image fiber.

In an embodiment of the mirror 2, the major axis is 25 mm long and the minor axis is 15 mm. It is narrower in width by 10 mm than a perfectly circular mirror having a diameter of 25 mm. But, this does not affect the quantity of light coming into the image fiber 11 and the broadness of the visual field.

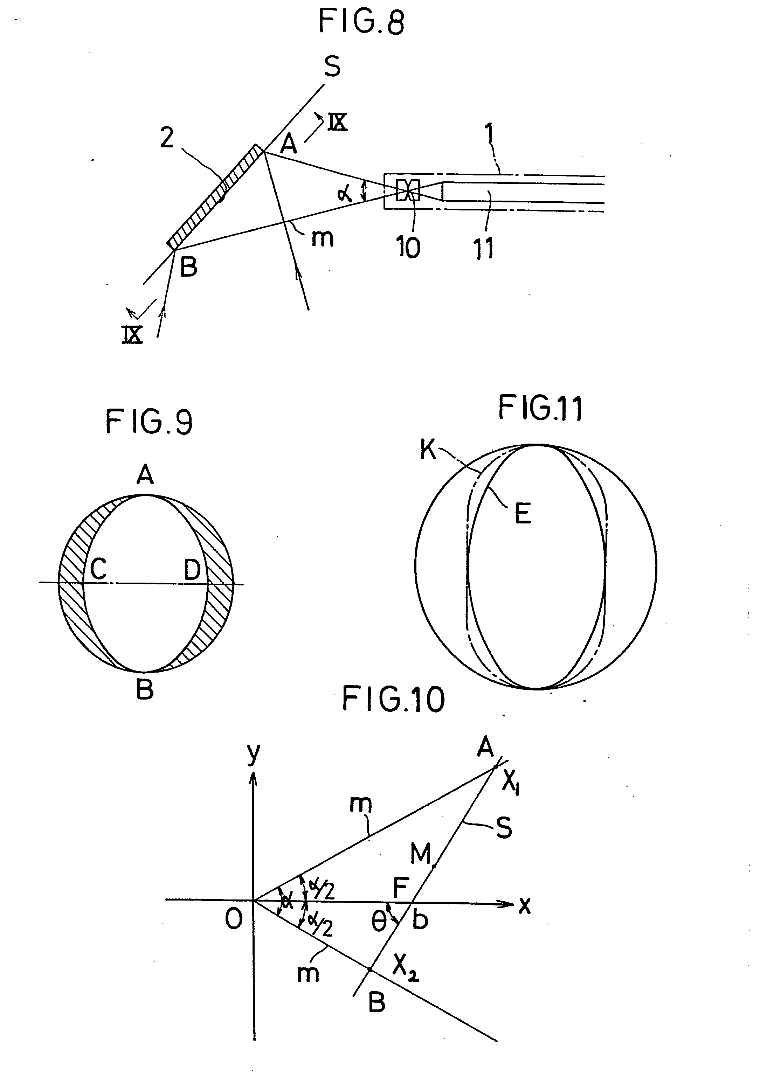

Referring to Fig. 8, the probe 1 accommodates an image fiber 11 and lenses 10. The image fiber has a circular section. Therefore, only a conic portion of the light reflected by the mirror 2 is focussed by the lens assembly 10 into the end of the image fiber. In other words, only the light falling within a cone m having a vertical angle of ⋋ and its vertex disposed in the center of the lens assembly 10 comes into the image fiber.

The light reflected in the range between A and B in the vertical section of the mirror is utilized. In the direction perpendicular to AB, however, the light reflected in a narrower range falls within the cone m.

Fig. 9 illustrates the mirror surface. The blank area falls within the cone m, while the hatched area does not fall. The area falling within the cone is in the form of an elongated circle having a major axis AB and a minor axis CD.

The hatched area is an area which cannot be utilized as a mirror because the light reflected by this area is not focused within the end face of the image fiber. Therefore, the mirror may be in the form of an elongated circle ADBC. A mirror without the useless hatched area is narrower and thus is easily inserted into a confined portion.

The ideal shape of the mirror will be discussed below. In Fig. 8, the cone m of light has its vertex disposed in the lens assembly and has a vertical angle of $\alpha$. Now the shape of a curved line at which the cone m intersects the mirror surface S will be discussed below.

A cone can be expressed in a quadratic formula with coordinates x, y and z in a rectangular coordinate system. A plane can be expressed in a linear formula with coordinates x, y and z. Therefore, an equation representing the curved line at which the cone m intersects the plane S is obtained by solving simultaneous equations consisting of the above-mentioned quadratic and linear formulas. By eliminating any one of three variables from the simultaneous equations, a quadratic formula having the remaining two variables is obtained.

A quadratic equation represents an ellipse, a hyperbola or a parabola. In the present case, it is essential that the edge of a mirror be in the form of a closed curve, from which it will be understood that the curved line at which the cone m intersects the plane S takes the form of an ellipse.

An ellipse is defined when two of the three factors, i.e., the major and minor axes, the distance between foci, and the eccentricity are determined. Now the ratio of the major axis to the minor axis of the ellipse will be considered below.

Referring to Fig. 10, the cone m has its vertex disposed on the origin O and its axis falling on the axis X. The plane S is assumed to be parallel to the axis Z.

Now the length of the major axis AB of the ellipse is to be found. The plane S forms an angle of θ with the X axis. The middle point M of the major axis AB is the center of the ellipse. A perpendicular to the XY plane is drawn at the point M. This perpendicular intersects the cone m at points C and D. The line segment CD represents the minor axis.

The line OA can be expressed by

$$y = x \tan\frac{\alpha}{2} \tag{1}$$

The line OB can be expressed by

$$y = -x \tan\frac{\alpha}{2} \tag{2}$$

The line AB can be expressed by

$$y = ( x - b ) \tan\theta \qquad (3)$$

where $\underline{b}$ is the abscissa of the point F at which the line AB intersects the X axis.

From Eq. (1) and (3), the abscissa $x_1$ of the point A is

$$x_1 = \frac{b \tan\theta}{\tan\theta - \tan\frac{\alpha}{2}} \qquad (4)$$

From Eq. (2) and (3), the abscissa $x_2$ of the point B is

$$x_2 = \frac{b \tan\theta}{\tan\theta - \tan\frac{\alpha}{2}} \qquad (5)$$

Since the length of the major axis AB is

$$AB = ( x_1 - x_2 ) \sec\theta \qquad (6)$$

$$AB = \frac{2b \tan\theta \sec\theta \tan\frac{\alpha}{2}}{\tan^2\theta - \tan^2\frac{\alpha}{2}} \qquad (7)$$

$$CD = \frac{2b \tan\theta \tan\frac{\alpha}{2}}{\sqrt{\tan^2\theta - \tan^2\frac{\alpha}{2}}} \qquad (8)$$

Therefore, the ratio R of the length of major axis to the length of minor axis is

$$R = \frac{AB}{CD} = \frac{\sec\theta}{\sqrt{\tan^2\theta - \tan^2\frac{\alpha}{2}}} \qquad (9)$$

- 14 -

In accordance with the present invention, a reflex mirror is used which is not in a perfectly circular form, but in the form of an elongated circle.

The elongated circle as termed herein is used in a broader sense including an ellipse.

In Fig. 11, E designates an ideal ellipse, while K disignates an elongated circle circumsribing the ellipse E at both ends of the major and minor axes. Such an elongated circle cannot be expressed by a quadratic formula, but can be expressed by an equation made up by adding a term or terms of even-numbered degrees to an equation representing an ellipse.

Because the light is reflected in an even number of times, the image coming into the eye of a viewer is free from bilateral reversal. The object can be inspected in a magnified form. Precise inspection and diagnosis are possible.

Because a reflex mirror in the form of an elongated circle is employed, the present invention has the following advantages:

(1) A wide-angle visual field is afforded by a small reflex mirror.

(2) The mirror with a slender surface can be easily inserted into a confined portion. Especially it is suited for the inspection of an oral cavity.

The device in accordance with the present invention can be used for the following applications:

(1)  Inspection of an oral cavity, etc. in dentists' and other physicians' offices

(2)  Inspection of the interior of a gas pipe, a water pipe, a tube in a heat exchanger, etc.

(3)  Inspection of a confined portion in civil engineering and construction work, maintenance of equipment, etc.

what are claimed are:

1.     A device for observing pictures of an object comprising an image transmission line for transmitting an image,
an optical system for forming the image of the object
at the front end of said image transmission line, a reflex
mirror for reflecting the light from the object and directing
it toward said optical system, a flexible cable accommodating
said image transmission line, and an image receiving
means provided at an end of said image transmission line
for observing the image transmitted, said image transmission
line having an image fiber comprising a bundle of picture
element fibers.

2.     The device as claimed in claim 1, wherein said
reflex mirror is in the form of an elongeted circle having
a minor axis perpendicular to the axis of said image
transmission line.

3.     The device as claimed in claim 1, wherein a light
quide for illumination is provided so as to be parallel
to said image fiber.

4.     The device as claimed in claim 2, wherein the
ratio of the length of said major axis to that of said minor axis is equal to

$$\frac{\sec\theta}{\sqrt{\tan^2\theta - \tan^2\frac{\alpha}{2}}}$$

wherein $\theta$ is an angle formed between an extension of the axis of said image fiber and the surface of said reflex mirror, and $\alpha$ is the vertical angle of the conic portion of the light reflected by said reflex mirror.

5.     The device as claimed in claim 1, wherein the image of the object is reflected in an even number of times.

6.     The device as claimed in claim 5, wherein a reflex mirror is provided in said image receiving means.

7.     The device as claimed in claim 3, further comprising a stator removably mounted on the front end of said flexible cable to form a grip portion, said image fiber and said light guide having their front end open at the front end of said stator, and a rotor mounted on said stator so as to be turnable with respect to said stator and engageable at one of a plurality of angularly spaced positions and carrying said reflex mirror at its front end.

8.    The device as claimed in claim 7, wherein said stator is formed with an annular groove and a plurality of axial grooves arranged at angularly spacings, and said rotor is formed with a plurality of projections arranged at angularly spacings, said projections being engageable in said annular groove and alignable with said axial grooves, whereby permitting said rotor to get off said stator when said projections are aligned with said axial grooves.

9.    The device as claimed in claim 1, wherein said image receiving means include a TV camera and a TV monitor.

0122537

FIG.1

FIG.2

6

5

7

4

8

9

2   1   3

FIG.3

2   10   15   3

1   11

14   6

5

3   16

4   11   12   13

# FIG.4

# FIG.5

# FIG.6

# FIG.7

0122537

3/3

0122537

FIG.8

FIG.9

FIG.11

FIG.10

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-3 434 775 (N. GOSSELIN) <br> * Whole document * | 1,3 | G 02 B 23/08 <br> A 61 B 1/04 |
| Y | | 1-9 | |
| Y | US-A-2 028 430 (W. BADDORF et al.) <br> * Page 2, left-hand column, lines 41-43; figures 3,4 * | 1-4 | |
| Y | DE-C- 454 046 (O. HECKL) <br> * Page 1, left-hand column, lines 38-43; figures 1-3 * | 1-5 | |
| Y | US-A-3 599 630 (M. SATO et al.) <br><br> * Column 4, lines 20-36; figure 1 * | 1,3,5, 6 | |
| Y | US-A-4 140 364 (N. YAMASHITA et al.) <br> * Column 2, lines 40-56; figure 3 * | 1,3,5 | G 02 B 5/00 <br> G 02 B 23/00 <br> A 61 B 1/00 |
| Y | US-A-3 556 086 (T. GORDON) <br><br> * Column 4, lines 39-71; figure 2 * | 1,3,7, 8 | |
| | --- -/- | | |

TECHNICAL FIELDS SEARCHED (Int. Cl. ³)

The present search report has been drawn up for all claims

| Place of search <br> THE HAGUE | Date of completion of the search <br> 11-07-1984 | Examiner <br> POPINEAU G.J.P. |
|---|---|---|

EPO Form 1503. 03.82

## European Patent Office

### EUROPEAN SEARCH REPORT

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. 3) |
|---|---|---|---|
| Y | DE-A-3 045 162 (P. HEITLINGER) * Claim 1; figures 1,2 * | 1,3,9 | |
| Y | DE-A-2 505 798 (H. SCHNEIDER) * Page 2, lines 6-10; figure 1 * | 1,3,9 | |

TECHNICAL FIELDS SEARCHED (Int. Cl. 3)

The present search report has been drawn up for all claims

| Place of search THE HAGUE | Date of completion of the search 11-07-1984 | Examiner POPINEAU G.J.P. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82